# EUROPEAN PATENT APPLICATION

(11) **EP 4 052 755 A1**
(43) Date of publication of application: **07.09.2022**
(21) Application number: 21160121.6
(22) Date of filing: 02.03.2021
(51) Int. Cl.: A61N 1/05, A61N 1/362, A61N 1/375, A61N 1/39

(54) **INTRACARDIAC DEVICE AND FIXING METHOD THEREFOR**

(71) Applicant: BIOTRONIK SE & Co. KG, 12359 Berlin (DE)
(72) Inventor: WHITTINGTON, R. Hollis, Portland, OR 97202 (US); HUGHES, Devan, Tualatin, OR 97026 (US); AUSTIN, Eric, Portland, OR 97221 (US); MUESSIG, Dirk, West Linn, OR 97068 (US); TAFF, Brian M., Portland, OR 97217 (US); MIDGETT, Madeline Anne, Portland, OR 97213 (US)
(74) Representative: Biotronik Corporate Services SE

(57) **Abstract**

The invention refers to an intracardiac device for fixing at an anchoring region within the tissue of a patient's heart, wherein the intracardiac device comprises one electrode (24) attached at its distal end and a housing (20) having a longitudinal axis (28), wherein the housing (20) comprises a shell surface, wherein the shell surface comprises at least one array (30, 31, 32) consisting of a plurality of similar fixation elements (35, 40, 42) attached to and extending radially or inclined from the shell surface, wherein the fixation elements of all arrays are adapted such that after deployment at least a part of the fixation elements is in contact with the tissue in the anchoring region, fixes the intracardiac device at the anchoring region and counteracts any displacement of the intracardiac device from the anchoring region. The invention further comprises a sleeve comprising the at least one array and a system comprising the intracardiac device and the sleeve.

## Description

The present invention refers to an implantable intracardiac device, such as an implantable intracardiac pacemaker.

Active or passive intracardiac devices, for example implantable intracardiac pacemakers (also known as leadless pacemakers), are well known miniaturized medical devices which are entirely implanted into a heart chamber of a patient. Intracardiac pacemakers are used for patients who suffer from a bradycardia. Intracardiac devices apply electrical stimulation in the form of pulses to the heart in order to generate a physiologically appropriate heartrate (intracardiac pacemakers) and/or in the form of shocks for cardioversion or defibrillation in order to restore a more normal heart rhythm. Alternative or additional functions of intracardiac devices comprise providing other electrical or electromagnetic signals to the heart or its surrounding tissue and sensing electrical or electromagnetic signals or other physiological parameters of the heart and/or its surrounding tissue.

Intracardiac device fixation mechanisms are currently only used for ventricular implantation. As intracardiac pacemaker use is expanded to dual-chamber applications, specialized atrial fixation methods are also needed. Atrial anatomy dictates safe and reliable fixation approaches. The right atrium lateral wall and appendage are extremely thin and sparsely covered with pectinate muscle. In contrast, the septal and posterior right atrium walls are smooth without any pectinate muscles. Given the differences in tissue properties between the ventricle and the atrium, such as thickness and elasticity, known solutions that are intended for ventricular location would not provide safe and reliable tissue contact for the atrial application.

Document US 8,700,181 B2 discloses an intracardiac medical device for implantation comprising electrodes formed as fixation mechanism similar to a pair of large diameter double helices similar to screws with a positive deflection near a base at the distal end of the device. For fixation the double helices are screwed into the heart's wall. The purpose of this shape is to ease implantation of the device but rendering unscrewing of the device very difficult due to its firm adhering to the wall. Further, the distal ends of the double helices may have serrated edges that prevent the device from unscrewing out of the heart's chamber wall. Such a fixation mechanism seems not applicable to the thin atrial walls.

Another embodiment of an intracardiac medical device for implantation into one atrium discussed in this document comprises a stabilizing intracardiac device extension connected to the housing. The stabilizing extension includes a stabilizer arm and/or an appendage arm or an elongated body or loop member configured to be passively secured within the heart. The stabilizing extension is located at the end of the intracardiac device opposite the electrode. The appendage arm includes a distal end upon which an electrode is located. The electrode is a passive electrode that is configured to simply rest against a selected activation site or an active fixation electrode that is configured to be secured to the tissue at the activation site. Further, the stabilizer arm has a distal end that is positioned in the superior vena cava (SVC), wherein the stabilizer arm extends across the whole right atrium. In this position, the stabilizer arm applies a quite high force to the SVC wall leading to an increased risk of injury of the thin tissue of the heart and the vein in this region.

Accordingly, there is the need for implantable intracardiac devices for atrial use having a fixing mechanism / anchoring method that would ensure reliable electrode contact with the thin walled atrial tissue substrate, simple implantation and low manufacturing effort and costs.

Document US 2004/0147973 A1 discloses an intra cardiac pulse generator for implantation into a cardiac chamber or a portion of the cardiac chamber such as the atrial appendage. The pacemaker comprises a hermetically sealed housing and a shield which is resilient and expands in its deployed state to conform to the shape of the anatomic structure that is used to retain the intra cardiac pulse generator, e.g. the atrial appendage.

An implantable medical device is described in US 2018/0126179 A1 having a housing and expandable and elongated anchoring members extending from the housing, wherein the housing extends from the first end of the expandable anchoring members towards the second end of the expandable anchoring members. Further, the first end of the housing extends past ends of the expandable anchoring member. The device is deployed and positioned such that the cathode electrode of the housing engages the heart wall to sense signals from the heart and/or applies pace signals to the heart.

Accordingly, there is the need for implantable intracardiac devices for atrial use having a fixation mechanism / anchoring method that would provide stable positioning of the device without damaging the sensitive myocardial tissue, simple implantation and low manufacturing effort and costs.

The above problem is solved by an intracardiac medical device as defined in claim 1.

In particular, an intracardiac device for fixing at an anchoring region within the tissue of a patient's heart, for example in an atrial appendage, is provided, wherein the intracardiac device comprises one electrode attached at its distal end and a housing having a longitudinal axis, wherein the housing comprises a shell surface, wherein the shell surface comprises at least one array consisting of a plurality of similar fixation elements attached to and extending radially or inclined from the shell surface. The fixation elements of all arrays are adapted such that after deployment at least a part of the fixation elements contacts the tissue in the anchoring region, fixes the intracardiac device at the anchoring region, and counteracts any displacement of the intracardiac device from the anchoring region.

If there are at least two arrays of fixation elements, the at least two arrays may comprise the same or different fixation element types. Such embodiment may be used in order to adapt the structure of the fixation mechanism to the anatomic conditions of the respective patient. For example, one array located most distally may be fabricated from a polymer to passively anchor the device, and one or more other arrays located proximally of the most distal array may have larger geometries and be made of a super elastic material, allowing them to expand and support to an increasing diameter chamber.

According to the invention, it means that similar fixation elements are formed by the same fixation element type having the same general form, such as described below: straight finger with hook, paddle or scale-like form. The fixation elements of the same type of the same array may be identical or similar in the sense that the length may be different or a part of the form such as the inclination of the end section forming the hook (see below).

One array may have at least two fixation elements within one cm² arranged in a pre-defined arrangement altogether forming a regular/irregular pattern at the shell surface of the housing. One array may at least comprise two rows, particularly within the length of the intracardiac device, e.g. 25 mm.

The above intracardiac device is especially suitable to be fixed in an atrial appendage. An implant located there does not interfere significantly with the blood flow to the respective ventricle. Also, it has been shown that closing off the appendage (mostly left atrial site) is a safe and effective procedure in patients with permanent atrial fibrillation. The atrial appendage may be the right atrial appendage or the left atrial appendage. The device is positioned such that the distal tip of the device is located in the back end or rearward end of the appendage. There, the at least one array distributes the pressure to the atrial myocardium as much as possible in order to provide stable positioning of the device without damaging the myocardial tissue. In particular, the pressure distribution is provided by the plurality of fixation elements of the at least one array, which are supported by the tissue in the anchoring region surrounding the intracardiac device. The intracardiac device according to the invention does not damage the atrium unnecessarily by avoiding deep penetration of the tissue by anchoring using the contact points with the heart wall and thereby does not cause unacceptable loss of blood. This is because the fixation elements are distributed in space over the shell surface of the housing/device avoiding force concentration at the tip of the device where the stimulation is being delivered.

Further, the fixation elements may be deformable from a pre-delivery state to an anchored state in the case the fixation element is flexible.

The intracardiac device according to the invention provides an atraumatic fixation and is configurable in size and stiffness of its fixation elements in order to meet the anatomical condition of the respective patient, in particular with regard to the form and size of the atrial appendage. Further, the fixation elements do not interfere with the stimulation.

The above defined implantable intracardiac medical device, for example a leadless pacemaker, may comprise a cylindrical housing or a housing having the form of a truncated cone having the above mentioned longitudinal axis and the electrode projecting from the distal end of the housing, wherein the electrode may be pin-shaped and extending into the direction of the longitudinal axis. Further, a header assembly may be arranged at and attached to the distal end of the housing of the intracardiac device such that the electrode projects through the header assembly, i.e. a respective through-going or complete opening of the header assembly. The opening may be a central opening. The cylindrical housing comprises the electronics module having a processor, an energy source (e.g. a battery or coil (for wireless charging)) and, if applicable, a communication component such as an antenna. The processor may comprise a storage unit for data storage. The processor may be adapted to process signals determined from the patient's body or received from the surrounding environment and/or to produce signals for treatment of the patient's heart. Such signals may comprise electrical stimulation in the form of pulses in order to generate a physiologically appropriate heartrate, shocks for cardioversion or defibrillation in order to restore a more normal heart rhythm and/or other electrical or electromagnetic signals to the heart or its surrounding tissue. Such signals are transformed and transmitted by the electronics module and may be applied by the pin-shaped electrode to the heart or its surrounding tissue. The pin-shaped electrode is electrically connected to the electronics module and the energy source. The hermetically sealed housing may comprise biocompatible material and/or electrically conducting material, e.g. titanium, and may function as another electrode. In one embodiment, at the distal end of the intracardiac device the electrode is located at a central position, preferably at the longitudinal axis of the intracardiac device.

In one embodiment, the at least one array of fixation elements covers more than 1/2 of the shell surface area. In another embodiment the at last one array of fixation elements covers more than 3/4 of the shell surface area.

In one embodiment, each fixation element of one array is a straight finger extending radially with regard to the longitudinal axis from the shell surface wherein the straight finger forms a hook at its end section furthest away from the shell surface. This means that the first section, i.e. the section closest to the shell surface of the housing extends radially with regard to the shell surface or the longitudinal axis of the housing. And the end section furthest away from the shell surface forms the hook. The first section and the end section may be located directly adjacent to each other. In this embodiment the fixation element may be formed by a wire or filament, wherein the wire or filament may have a circular, elliptical or angular (e.g. rectangular or square) cross section. The diameter of the wire may be, for example, in the range of 0.04 mm to 0.7 mm.

In one embodiment, the bent end section of each fixation element forms the hook, wherein the end section of each hook points into proximal direction or perpendicular to this direction (i.e. runs along circumferential direction) and runs parallel to the direction of the longitudinal axis or inclined thereto. If the end section runs inclined to the longitudinal axis, the inclination may be provided within a plane running tangential to a circle line formed around the longitudinal axis at the bend and/or into radial direction with regard to the longitudinal axis. Further, the inclination of the end section may be similar for all fixation elements of the same array or different in a pre-defined way.

In one embodiment, each fixation element of one array is a flat paddle extending inclined from the shell surface. This embodiment further avoids impact to the tissue providing fixation within both a fine tissue structure, where many contact points are possible, and a tissue with more heterogeneity, where more force is applied across fewer contact points.

In one embodiment, each fixation element of one array has a scale-like form extending inclined from the shell surface. Due to their scale-like form the fixation elements overlap each other which support their contact to the surrounding tissue of the heart. One advantage of fixation elements having a scale-like form is that the diameter of the device introducer, e.g. a delivery catheter, may be minimized, while still providing support and contact to the tissue. Preferably, each fixation element having a scale-like form has width in the range of 0.5 mm to 5 mm, particularly in case of the device has a diameter of about 7 mm and comprises 3 to 40 circumferentially arranged fixation elements having the scale-like form.

In one embodiment, the fixation elements of the same array or of different arrays have different length. Such embodiment may be used in order to adapt the structure of the fixation mechanism to the anatomic conditions of the respective patient.

In one embodiment, the at least one array is fixed to a sleeve forming a covering layer of the shell surface of the housing. Such sleeve may be easily integrated into production as an add-on sleeve which may be fixed to the intracardiac device before implantation. The sleeve may consist of moldable material, such as, for example, polyurethane or silicone. Using a sleeve comprising the at least one array of fixation elements, which may be attached around the intracardiac device, allows to realize different configurations of the fixation dependent on the application type (atrium or ventricle) and patient anatomy (large atrial appendage or small one). Alternatively, the structure forming the at least one array consisting of a plurality of similar fixation elements may be patterned directly on the shell surface of the housing, e.g. by molding with a mold. The patterning may be used to electrically isolate an electrically conductive housing, particularly such that a proximal part of the housing is insulated from the distal (pacing) electrode and may act as return electrode for the pacing electrode. Preferably the proximal part of the housing acting as return electrode has the form of a ring, thus forming a ring electrode. Preferably, the sleeve may be characterized by a thickness in the range of 0.125 mm to 0.75 mm, particularly the device has a diameter of about 7 mm.

In one embodiment, the plurality of arrays is attached to the shell surface of the housing such that they are accommodated in an offset arrangement (if seen in the direction of the longitudinal axis) at the circumference of the housing. Thereby, a patient-adapted distribution of forces may be provided. In this embodiment, each of these arrays does not extend over the full circumference of the shell surface but covers, for example, between 60 % and 90 % of the circumference.

In one embodiment, the fixation elements of the same array are arranged in a row and/or in a column and/or offset to one another along one direction at the shell surface of the housing. Again, such different arrangement of fixation elements may allow the adaption of the fixation mechanism to the specific needs of the patient and the intracardiac device.

In one embodiment, the intracardiac device further comprises a hitch at the proximal end of the housing. The hitch is used to deploy and retrieve the intracardiac device. The hitch has a curved proximal end so that a catheter structure may interact with the hitch, for example pull the hitch. Preferably, the hitch has a neck with a diameter smaller than the outer diameter of the device housing. This neck expands into a "T-shaped" profile with a channel to accommodate a tether within the implantation tool so that a catheter structure may secure and interact with the intracardiac device during release and resheathing. For example, the catheter may push and pull the hitch in and out of the catheter. Once released, the "T-shape" allows for a snare of a retrieval catheter to recapture the device if a removal is required.

The problem is also solved by a sleeve for the intracardiac device described above, wherein the sleeve is attachable to the shell surface of the housing and the at least one array consisting of a plurality of similar fixation elements is attached to and extends radially or inclined from the sleeve, wherein the fixation elements of all arrays are adapted such that after deployment of the intracardiac device covered at its shell surface with the intracardiac device at least a part of the fixation elements is in contact with the tissue in the anchoring region, fixes the intracardiac device at the anchoring region and counteracts any displacement of the intracardiac device from the anchoring region and a system comprising the intracardiac device and the sleeve. The sleeve and the system have the properties and advantages described with regard to the intracardiac device and the sleeve above.

In one embodiment, the fixation elements and/or the sleeve comprises a biocompatible material, for example a metal or a plastic material, e.g. silicone or other flexible polymer such as polyurethane, and/or a shape memory material such as Nitinol. Alternatively, only parts of the fixation elements or the whole fixation element is made of such material.

The present invention will now be described in further detail with reference to the accompanying schematic drawing, wherein
- Fig. 1: shows a one embodiment of an inventive intracardiac device in a side view,
- Fig. 2: depicts a second embodiment of an inventive intracardiac device in a side view,
- Fig. 3: shows the embodiment of Fig. 1 in a deployed state from the side,
- Fig. 4: shows the embodiment of Fig. 1 in a top view,
- Fig. 5: shows a further embodiment of an inventive intracardiac device in a side view, and
- Fig. 6: shows a further embodiment of an inventive intracardiac device in a deployed state from the side.

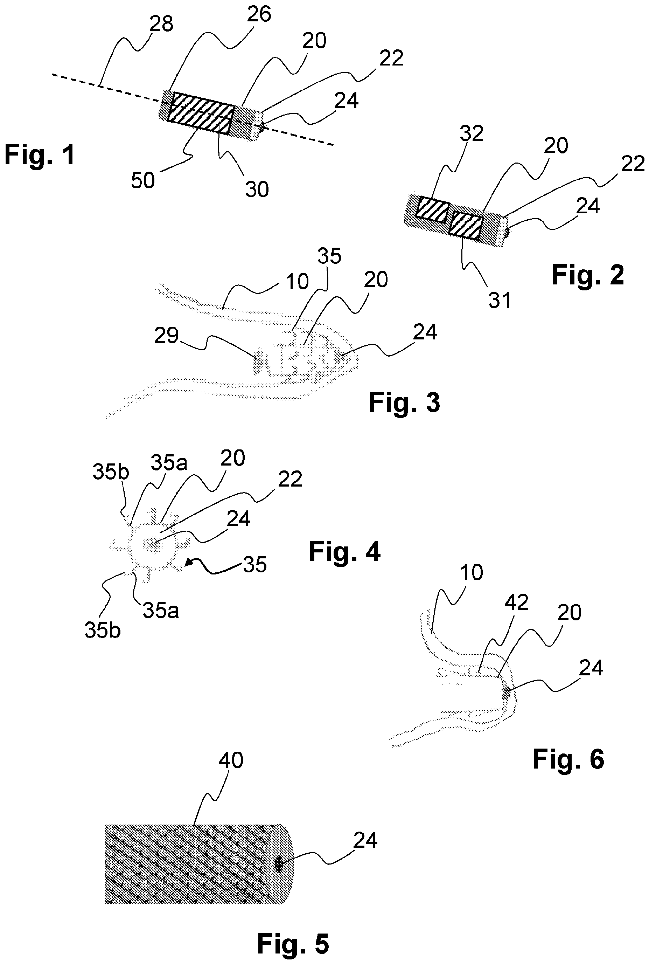

Fig. 1 illustrates a first embodiment of an implantable intracardiac device, e.g. a leadless pacemaker. The intracardiac device comprises a cylindrical housing 20. A header 22 and a pin-shaped electrode 24 are located at the distal end of the housing 20. The pin-shaped electrode 24 protrudes through the header 22 and from its distal end face. The cylindrical housing 20 has a longitudinal axis 28, which axis also forms the axis of the pin-shaped electrode 24.

The housing 20 of the intracardiac device contains a battery and an electronic module comprising a processor and ensures, hermetically sealing of these components. Battery and electronic module are electrically connected to the electrode 24 and provide (together with electrode 24) the electrical stimulation of the heart or processing of electrical signals determined from the heart. Further, the housing 20 may contain components for communication such as an antenna.

The housing 20 further comprises at its shell surface an array 30 consisting of a plurality of similar fixation elements attached to and extending radially or inclined from the shell surface. The array 30 is attached to one section of the shell surface of the housing and runs along the whole circumference in this section. The fixation elements of this array 30, which are not shown in detail in Fig. 1, are adapted such that after deployment at least a part of the fixation elements is in contact with the tissue in the anchoring region, fixes the intracardiac device at the anchoring region and counteracts any displacement of the intracardiac device from the anchoring region. The array 30 may be arranged and/or attached to a sleeve 50 forming a circumferential covering layer of the shell surface of the housing 20. Preferably, the sleeve 50 is made of a biocompatible material, for example a metal or a plastic material, e.g. silicone or other flexible polymer such as polyurethane, and/or a shape memory material such as nitinol.

Fig. 2 depicts an alternative embodiment having two arrays 31, 32 fixed to a section of the shell surface of the housing 20, but each array does not cover the whole circumference of the shell surface. The two arrays 31, 32 are arranged with an offset with regard to the circumference of the shell surface as shown in Fig. 2. The arrays 30, 31, 32 are shown schematically in Fig. 1 and 2, the structure of the fixation elements is not shown in detail but the area in which the fixation elements are fixed to the shell surface of the housing 20.

The array 30 covers more than half of the area of the shell surface of the housing 20 and the arrays 31, 32 together cover 3/4 of the area of the shell surface of the housing 20.

Figs. 3 and 4 illustrate the accommodation of single fixation elements 35 at the shell surface of the embodiment of Fig. 1 in a deployed state after fixing the device at an anchoring position within the heart's tissue of a patient, e.g. the right atrial appendage. The electrode 24 is located at or near the end of the atrial appendage. The wall of the heart at the right atrial appendage is provided with reference number 10. Further, it is shown that at the proximal end of the housing 20, the intracardiac device comprises a hitch 29 for deployment and retrieval.

Each fixation element 35 formed from a wire comprises a first section 35a forming a straight finger extending radially from the shell surface of the housing 20 (see Fig. 4). Further, at the fixation element 35 is a second (end) section 35b bent from the first section 35a forming a hook. As one can derive from Fig. 4 the first section 35a and the second section 35b form approximately a right angle. However, as one can derive from Fig. 4, the second sections 35b of fixation elements 35 within one row (at the circumference) have a different orientation within the respective tangential plane which one can draw at the end of the first section of each fixation element 35 with regard to the circumference at this position. Some of the second sections 35b are directed partly to the adjacent fixation element within the same row (i.e. partly extend into the direction of the circumference), some are rotated such that they are partly directed into the direction of the longitudinal axis or fully into the direction of the longitudinal axis.

The fixation elements 35 may consist of nitinol wires and may have a cross section diameter in the range of 0.04 mm to 0.7 mm.

The fixation elements 35 may be welded to the shell surface of the housing 20 of the intracardiac device. However, also other attachment mechanisms are conceivable.

The embodiment of an intracardiac device shown in Fig. 5 comprises overlapping fish-scale fixation elements 40 attached to the whole shell surface of the housing 20.

Fig. 6 shows an embodiment where the fixation elements are formed as flat paddles 42.

The above intracardiac device provides a reliable fixing for an intracardiac device within an atrial wall of the heart, in particular an atrial appendage, or other thin walls of the heart as the acting forces are distributed over a large area by the at least one array of fixation elements. The proposed intracardiac device allows easy usage and is associated with low manufacturing costs.

## Claims

1. An intracardiac device for fixing at an anchoring region within the tissue of a patient's heart, wherein the intracardiac device comprises one electrode (24) attached at its distal end and a housing (20) having a longitudinal axis (28), wherein the housing (20) comprises a shell surface, wherein the shell surface comprises at least one array (30, 31, 32) consisting of a plurality of similar fixation elements (35, 40, 42) attached to and extending radially or inclined from the shell surface, wherein the fixation elements of all arrays are adapted such that after deployment at least a part of the fixation elements is in contact with the tissue in the anchoring region, fixes the intracardiac device at the anchoring region and counteracts any displacement of the intracardiac device from the anchoring region.

2. The intracardiac device according to claim 1, wherein the at least one array (30, 31, 32) of fixation elements covers more than 1/2 of the shell surface area.

3. The intracardiac device according to any of the previous claims, wherein each fixation element (35) of one array is a straight finger (35a) extending radially with regard to the longitudinal axis from the shell surface wherein the straight finger forms a hook at its end section (35b) furthest away from the shell surface.

4. The intracardiac device according to claim 3, wherein a bent end section (35b) of each fixation element forms the hook, wherein the end section of each hook points into proximal direction and runs parallel to the direction of the longitudinal axis (28) or inclined thereto.

5. The intracardiac device according to any of claims 1 and 2, wherein each fixation element of one array is a flat paddle (42) extending inclined from the shell surface.

6. The intracardiac device according to any of claims 1 and 2, wherein each fixation element of one array has a scale-like form (40) extending inclined from the shell surface.

7. The intracardiac device according to any of the previous claims, wherein the fixation elements of the same array or of different arrays have different length.

8. The intracardiac device according to any of the previous claims, wherein the at least one array is fixed to a sleeve (50) forming a covering layer of the shell surface of the housing.

9. The intracardiac device according to any of the previous claims, wherein the plurality of arrays are attached to the shell surface of the housing such that they are accommodated in an offset arrangement at the circumference of the housing.

10. The intracardiac device according to any of the previous claims, wherein the fixation elements of the same array are arranged in a row and/or in a column and/or offset to one another along one direction at the shell surface of the housing.

11. A sleeve (50) for the intracardiac device according to any of the claims 8 to 10, wherein the sleeve (50) is attachable to the shell surface of the housing and the at least one array consisting of a plurality of similar fixing elements is attached to and extends radially or inclined from the sleeve (50), wherein the fixing elements of all arrays are adapted such that after deployment of the intracardiac device covered at its shell surface with the intracardiac device at least a part of the fixing elements is in contact with the tissue in the anchoring region, fixes the intracardiac device at the anchoring region and counteracts any displacement of the intracardiac device from the anchoring region.

12. A system comprising the intracardiac device according to any of the claims 8 to 10 and the sleeve (50) according to claim 11.
